Europäisches Patentamt

European Patent Office

Office européen des brevets

(19)

(11) **EP 0 550 031 B1**

(12) **EUROPÄISCHE PATENTSCHRIFT**

(45) Veröffentlichungstag und Bekanntmachung des Hinweises auf die Patenterteilung:
**08.05.1996 Patentblatt 1996/19**

(51) Int. Cl.$^6$: **A61K 9/00**, A61K 9/12

(21) Anmeldenummer: **92121985.3**

(22) Anmeldetag: **24.12.1992**

(54) **Medizinische Aerosolformulierung**

Aerosol compositions

Compositions d'aérosol

(84) Benannte Vertragsstaaten:
**AT BE CH DE DK ES FR GB IT LI NL SE**

(30) Priorität: **31.12.1991 DE 4143263**

(43) Veröffentlichungstag der Anmeldung:
**07.07.1993 Patentblatt 1993/27**

(73) Patentinhaber: **HOECHST AKTIENGESELLSCHAFT
D-65926 Frankfurt am Main (DE)**

(72) Erfinder:
• **Deger, Hans-Matthias, Dr.
W-6238 Hofheim/Ts. (DE)**
• **Schütz, Claudia
W-6093 Flörsheim am Main (DE)**

(56) Entgegenhaltungen:
**EP-A- 0 372 777          EP-A- 0 384 371
EP-A- 0 504 112          EP-A- 0 518 600**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft medizinische Aerosolformulierungen, die frei von Fluorchlorkohlenwasserstoffen sind.

Medizinische Aerosolformulierungen sind insbesondere zur bukkalen, nasalen und topischen Verabreichung geeignet. Besondere Bedeutung haben sie z.B. bei der Verabreichung von Bronchodilatatoren über die Atemwege der Patienten erlangt.

Im allgemeinen enthalten medizinische Aerosolformulierungen den Wirkstoff, oberflächenaktive Stoffe und ein druckverflüssigtes Gas als Treibmittel. Sie werden in einem Druckbehälter, meist aus Metall oder Glas, der mit einer Ventileinrichtung zur Entnahme des Inhalts versehen ist, in den Handel gebracht. Die oberflächenaktiven Stoffe dienen zur Herstellung einer beständigen Suspension von gleichmäßig verteilten feinen Wirkstoffteilchen in dem Treibmittel und gleichzeitig zur Schmierung der Ventileinrichtung.

Als Treibmittel dienen druckverflüssigte Inertgase. Bei medizinischen Aerosolformulierungen wurden bisher meistens Gemische von Trichlorfluormethan (R 11) und Dichlordifluormethan (R 12) oder von Dichlordifluormethan (R 12) und 1,2-Dichloretrafluorethan (R 114) verwendet. Diese Mischungen wurden wegen ihrer Unbrennbarkeit, chemischen und physikalischen Stabilität, weitgehenden physiologisch/toxikologischen Unbedenklichkeit und günstigen Treib- und Lösemitteleigenschaften eingesetzt. Je nach der Zusammensetzung der Mischungen konnten die gewünschten Drücke, Dichten, Viskositäten und Stabilität der Aerosolformulierungen eingestellt werden.

Aufgrund ihrer hohen Beständigkeit gegen biotischen und abiotischen Abbau gelangen diese in die Atmosphäre abgegebenen Fluorchlorkohlenwasserstoffe ungehindert in die Stratosphäre, wo sie durch die harte solare UV-Strahlung photolysiert werden und Chlor-Radikale verlieren. Diese Chlor-Radikale können mit dem Ozon in einer komplexen Reaktionsfolge reagieren und so zu einer Schwächung der als UV-Strahlenschutzschirm wirkenden Ozonschicht führen. Daher sucht man nach geeigneten Treibmitteln, die nicht in der Lage sind, Ozon abzubauen, aber dennoch die weiter oben genannten günstigen Eigenschaften der bisher verwendeten Fluorchlorkohlenwasserstoffe haben.

In der EP-A- 0 384 371 werden Treibmittel für Aerosole beschrieben, die aus druckverflüssigtem 2-Hydroheptafluorpropan oder dessen Gemisch mit druckverflüssigtem Propan und/oder n-Butan und/oder i-Butan und/oder Dimethylether und/oder 1,1-Difluorethan bestehen. Als Anwendungsgebiete für diese Treibmittel werden Aerosol-Produkte, die nur diese Treibmittel enthalten und z.B. zum Reinigen von Kamaralinsen verwendet werden, sowie Aerosolprodukte genannt, die diese Treibmittel zusammen mit einem Füllprodukt enthalten, wie Insektizidspray, Raumspray, Deodorantspray, Parfümspray, Shampoo, Duschschaum, Rasierschaum, Sonnenschutzschaum. In Beispielen für ein Körperdeodorantspray und ein Parfümspray enthält das Füllprodukt als Lösemittel für die Wirkstoffe Ethanol.

Bei medizinischen Aerosolen ist der Ersatz der bisher verwendeten Fluorchlorkohlenwasserstoffe besonders kritisch. Zum einen müssen die Ersatztreibmittel physiologisch/toxikologisch unbedenklich sein. Zum anderen sollen sie möglichst bezüglich Unbrennbarkeit, Druck, Dichte, Viskosität, Verträglichkeit und Stabilisierung der Wirkstofformulierung den bisher verwendeten Treibmitteln ähnlich sein, so daß die bisherigen Wirkstofformulierungen, deren physiologische Verträglichkeit nachgewiesen ist, unverändert benutzt werden können. Ferner müssen sie mit den Elastomeren der Ventileinrichtungen gut verträglich sein.

Aus der EP-A- 0 372 777 sind medizinische Aerosolformulierungen bekannt, bei denen als Treibmittel 1,1,1,2-Tetrafluorethan (R 134a) verwendet wird, dem wenigstens eine Verbindung mit höherer Polarität als 1,1,1,2-Tetrafluorethan zugesetzt wird. Als Verbindungen mit höherer Polarität als 1,1,1,2-Tetrafluorethan werden Alkohole, wie Ethanol, Isopropanol und Propylenglykol, Kohlenwasserstoffe, wie Propan, Butane und Pentane, andere Treibmittel aus der Gruppe der Fluorchlorkohlenwasserstoffe und 1,2-Difluorethan (R 152a) sowie Dimethylether genannt. Von diesen Adjuvantien wird angegeben, daß sie zusammen mit R 134a ein Treibmittelsystem ergeben, das vergleichbare Eigenschaften hat wie die auf Fluorchlorkohlenwasserstoffen basierenden Treibmittelsysteme, so daß die bisher in pharmazeutischen Formulierungen verwendeten oberflächenaktiven Stoffe und Additive sowie konventionellen Ventilkomponenten verwendet werden können. Zu der Wirkungen der Adjuvantien wird im einzelnen auf die Möglichkeit der Druckerniedrigung und -erhöhung, der Modifizierung der Dichte, Viskosität und Grenzflächenspannung hingewiesen. Die alleinige Verwendung von R 134a ohne Adjuvantien wird als ungeeignet beschrieben.

Aufgabe der vorliegenden Erfindung ist die Bereitstellung einer medizinischen Aerosolformulierung mit einem verbessertem Treibmittelsystem.

Zur Lösung dieser Aufgabe wird eine medizinische Aerosolformulierung vorgeschlagen, die wenigstens einen medizinischen Wirkstoff, druckverflüssigtes Heptafluorpropan sowie wenigstens einen oberflächenaktiven Stoff, ausgenommen monoacetylierte oder diacylierte Monoglyceride, enthält.

Heptafluorpropan (R 227), unter dem sowohl 2-Hydroheptafluorpropan als auch 1-Hydroheptafluorpropan zu verstehen ist, ist überraschenderweise wesentlich geeigneter als R 134a zum Ersatz der bisher häufig in medizinischen Aerosolformulierungen verwendeten Treibmittelgemische R 11/12 und R 12/114.

So zeigt R 227 verglichen mit R 134a einen wesentlich günstigeren Dampfdruckverlauf. R 134a hat bei 50°C einen Dampfdruck von 13,2 bar, der über den von den Richtlinien der Technischen Regeln für Druckgase 300 geforderten 12 bar für Druckgaspackungen liegt. Für bis zu 12 bar sind z.B. die überwiegend eingesetzten Aluminiumdosen zugelassen.

Schon aus diesem Grund kann R 134a nur in Kombination mit Drucksenkern eingesetzt werden. Demgegenüber hat R 227 bei 50°C einen Dampfdruck von 9,2 bar, so daß Drucksenker nicht erforderlich sind. R 227 hat außerdem Druck-/Dichteverläufe, die mit denen der bisher verwendeten Treibmittelgemische weit mehr übereinstimmen als das bei R 134a der Fall ist. Ferner ist teilweise die Elastomerverträglichkeit sowie die Lösewirkung für die bei medizinischen Aerosolen einsetzbaren oberflächenaktiven Stoffe besser und folglich auch die Stabilität der Formulierungen.

Dies alles ermöglicht den Austausch der bisher verwendeten Treibmittelgemische durch das unbrennbare R 227 ohne größere Änderung der bisherigen medizinischen Aerosolformulierungen, so daß die erprobten Wirkstoff/oberflächenaktive Stoffe-Kombinationen beibehalten werden können.

Der erfindungsgemäßen Aerosolformulierung können Verbindungen mit höherer Polarität als R 227 zugesetzt werden. Als geeignete Verbindungen kommen niedere Kohlenwasserstoffe wie Propan, Butane und Pentane, aliphatische Ether, wie Dimethylether und aliphatische Alkohole in Frage. Vorzugsweise werden einwertige Alkohole mit niedrigem Siedepunkt und hoher Flüchtigkeit verwendet. Besonders bevorzugt sind Ethanol und Isopropanol.

Diese Verbindungen werden dann zugesetzt, wenn der Druck herabgesetzt werden soll. Da aus Kostengründen manchmal anstelle von Aluminiumdosen Weißblechdosen eingesetzt werden, deren Zulassung auf einen Druck von 8 bar bei 50°C beschränkt ist, ist es erforderlich, den Druck zu senken. Aber auch wenn eine Erniedrigung des Druckes nicht erforderlich ist, ist es bevorzugt diese Verbindungen zuzusetzen. Sie dienen als Lösevermittler für die Löslichkeit von oberflächenaktiven Stoffen in R 227 oder dessen Mischungen mit anderen Treibmitteln, so daß die Stabilität der Formulierungen häufig noch zusätzlich erhöht werden kann.

Das Gewichtsverhältnis von R 227 zu der Verbindung mit der höheren Polarität wird so gewählt, daß der Druck in dem die Aerosolformulierung enthaltenden Druckbehälter bei Raumtemperatur vorzugsweise nicht unter 3 bar fällt. Bei Verwendung der Alkohole, insbesondere Ethanol und Isopropanol, beträgt das Gewichtsverhältnis von R 227 zu Alkohol mindestens 70 : 30. Besonders bevorzugt liegt das Gewichtsverhältnis im Bereich von 85 : 15 bis 96 : 4.

Die erfindungsgemäße Aerosolformulierung kann außer R 227 ein weiteres Treibmittel enthalten. Als geeignete Treibmittel kommen insbesondere Fluorkohlenwasserstoffe aus der Gruppe $C_aH_bF_c$ in Frage, wobei b + c = 2a + 2 ist, und a 1 bis 3, b 0 bis 4 und c 2 bis 8 bedeutet. Beispiele für besonders geeignete Treibmittel aus dieser Gruppe sind Difluormethan (R 32), Pentafluorethan (R 125), 1,1,2,2-Tetrafluorethan (R 134), 1,1,2-Trifluorethan (R 143), 1,1,1-Trifluorethan (R 143a), 1,1-Difluorethan (R 152a), Oktafluorpropan (R 218), Hexafluorpropan (R 236) und Pentafluorpropan (R 245). Diese Treibmittel können in beliebigen Mengen zugesetzt werden, solange Dampfdruck- und Dichteverlauf von R 227 oder R 227 in Kombination mit den oben genannten Verbindungen mit höherer Polarität als R 227 nicht wesentlich geändert werden. Vorzugsweise enthält die erfindungsgemäße Aerosolformulierung 50 bis 95 Gew.-% Heptafluorpropan.

Die erfindungsgemäßen Aerosolformulierungen können als medizinische Wirkstoffe z.B. Betamimetika, Steroide, Gromoglycide, Nedocromil, Anticholinergika, Antihistaminika, Kalium - Kanalöffner, z.B. vom Cromantyp, Rezeptorantagonisten z.B. Bradykininantagonisten und Chlorid - Kanalmodulatoren, z.B. Furosemid, enthalten. Der Gehalt des medizinischen Wirkstoffs in der erfindungsgemäßen Aerosolformulierung liegt in der Regel im Bereich von 0,01 bis 5 Gew.-%. Bevorzugt liegt er im Bereich von 2 bis 5 Gew.-%. Die Teilchengröße der Wirkstoffe liegt in der Regel unter 20 μm und sollte möglichst klein sein, vorzugsweise unter 6 μm.

Die erfindungsgemäßen Aerosolformulierung enthalten als oberflächenaktive Stoffe solche die toxikologisch unbedenklich sind. Nur beispielsweise wird auf einige der üblichen in Frage kommenden Stoffe hingewiesen, wie Polyoxyethylensorbitan-fettsäureester, z.B. Polyoxyethylen(20)sorbitan-monolaurat und Polyoxyethylen(20)sorbitan-monooleat, Sorbitan-fettsäureester, z.B. Sorbitan-trioleat, Glycerin-fettsäureester, Ölsäure, Polyalkylenglykol auf Basis von Ethylenoxid und Propylenoxid, Quartäre Ammoniumverbindungen vom Typ des Alkylbenzyldimethylammoniumchlorids mit Alkylresten von $C_8H_{17}$ bis $C_{18}H_{37}$ und Lezithine wie z.B. Lezithin-Epikuron. Üblicherweise beträgt der Gehalt an oberflächenaktiven Stoffen in den erfindungsgemäßen Aerosolformulierungen bis 5 Gew.-%. Die Menge hängt von dem oberflächenaktiven Stoff, dem jeweiligen Wirkstoff sowie der jeweiligen Wirkstoffmenge in der Formulierung ab.

Die Erfindung wird an Hand der Figuren und Beispiele näher erläutert.

Es zeigen:

Fig. 1    die Dichte in Abhängigkeit von der Temperatur für zwei bisher in medizinischen Aerosolformulierungen verwendete Fluorchlorkohlenwasserstoffgemische R 11/12 und R 12/114, für R 134a sowie für das erfindungsgemäß eingesetzte R 227;

Fig. 2    den Gasdruck in Abhängigkeit von der Temperatur für dieselben druckverflüssigten Gase bzw. Gasgemische wie in Fig. 1 sowie zusätzlich für R 12;

Fig. 3    den Druck in Abhängigkeit vom Mischungsverhältnis von Ethanol und druckverflüssigtem R 134a bei 20°C und 50°C;

Fig. 4    den Druck in Abhängigkeit vom Mischungsverhältnis von Ethanol und dem erfindungsgemäß eingesetztem druckverflüssigtem R 227 bei 20°C und 50°C;

Fig. 5    den Druck in Abhängigkeit vom Mischungsverhältnis von Isopropanol und druckverflüssigtem R 134a bei 20°C und 50°C;

3

Fig. 6  den Druck in Abhängigkeit vom Mischungsverhältnis von Isopropanol und dem erfindungsgemäß eingesetzten druckverflüssigtem R 227 bei 20°C und 50°C.

Bei den Messungen der in den Figuren gezeigten Kurven und in den Beispielen wurde jeweils 2-Hydroheptafluorpropan als R 227 verwendet.

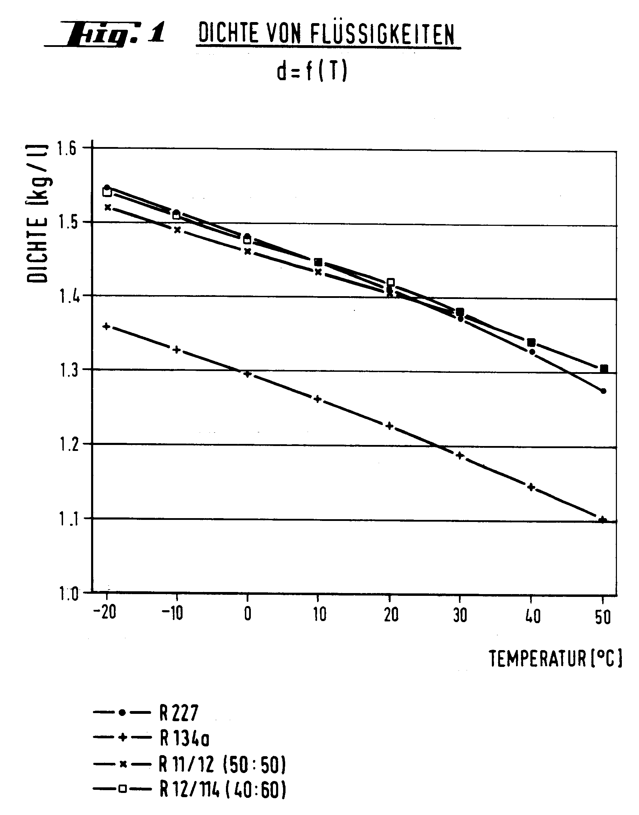

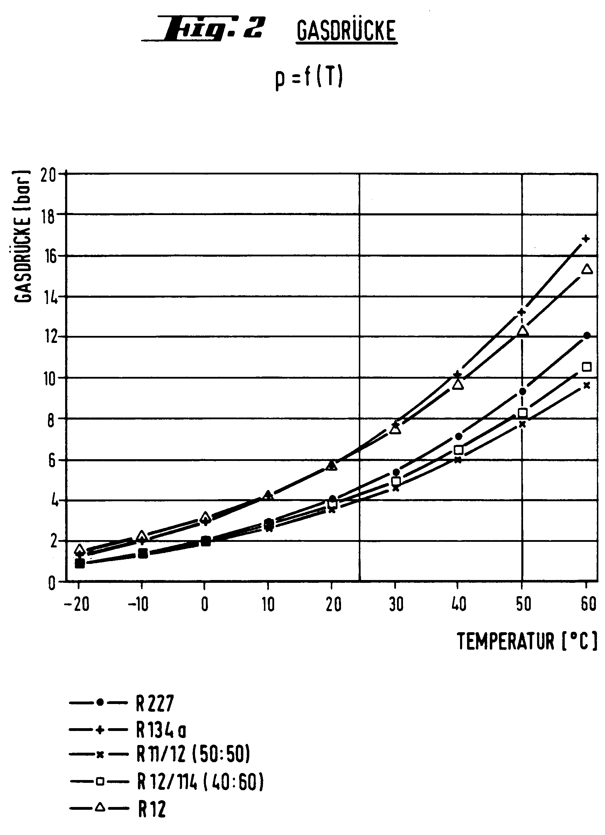

Die Figuren 1 und 2 zeigen, daß die Druck-/Dichtekurven von R 227 einen mit den bisher eingesetzten Fluorchlorkohlenwasserstoffgemischen weit mehr übereinstimmenden Verlauf aufweisen als R 134a.

Beispiel 1

Es wurde die Elastomerverträglichkeit durch Kaltextraktion nach DIN 8944 für R 134a und R 227 untersucht. Die Ergebnisse sind in folgender Tabelle 1 zusammengestellt.

Tabelle 1

| Polymer | R 134a | | R 227 | |
|---|---|---|---|---|
| | Massenänderung (%) | Längenänderung (%) | Massenänderung (%) | Längenänderung (%) |
| Butylkautschuk | -1,1 | 0 | -0,6 | 0 |
| Acrylnitrilbutadienkautschuk | -0,4 | 0 | -0,2 | 0 |
| Chloroprenkautschuk | -0,6 | 0 | -0,4 | 0 |
| Fluorkautschuk | 7,3 | 10,8 | 2,3 | 0,5 |
| Naturkautschuk | -0,6 | 0 | -0,4 | 0 |
| Polyamid | 0 | 0,1 | 0 | 0,16 |
| Diese Werte zeigen, daß die Elastomerverträglichkeit von R 227 zum großen Teil besser ist als die von R 134a. | | | | |

Beispiel 2

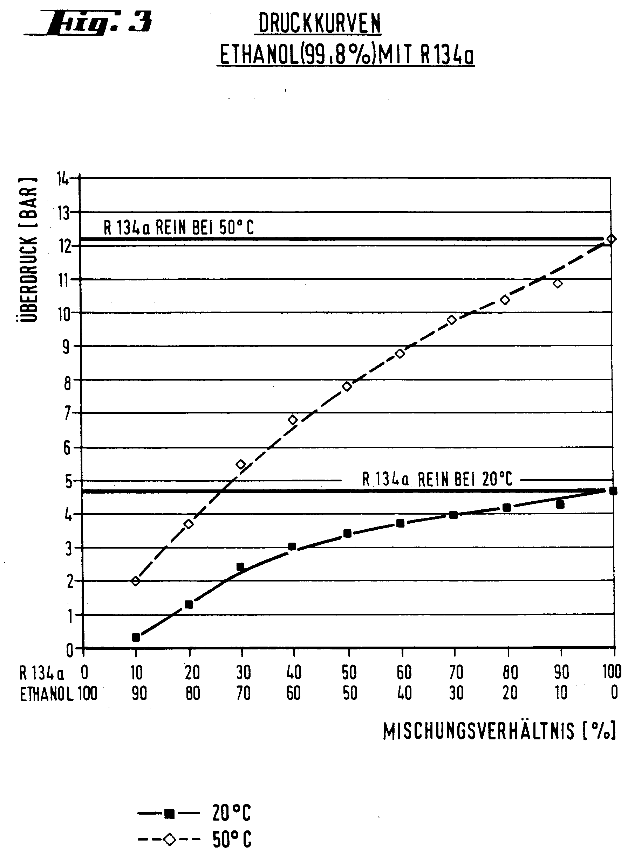

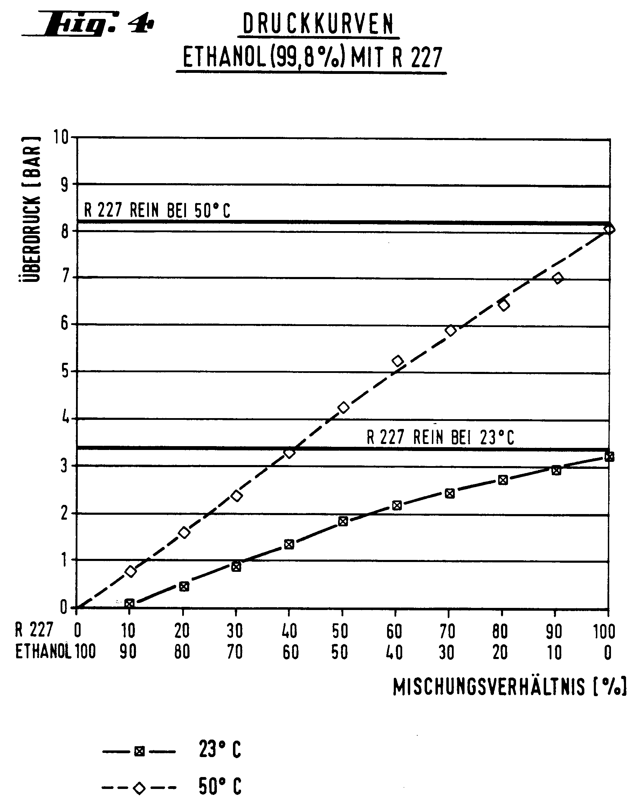

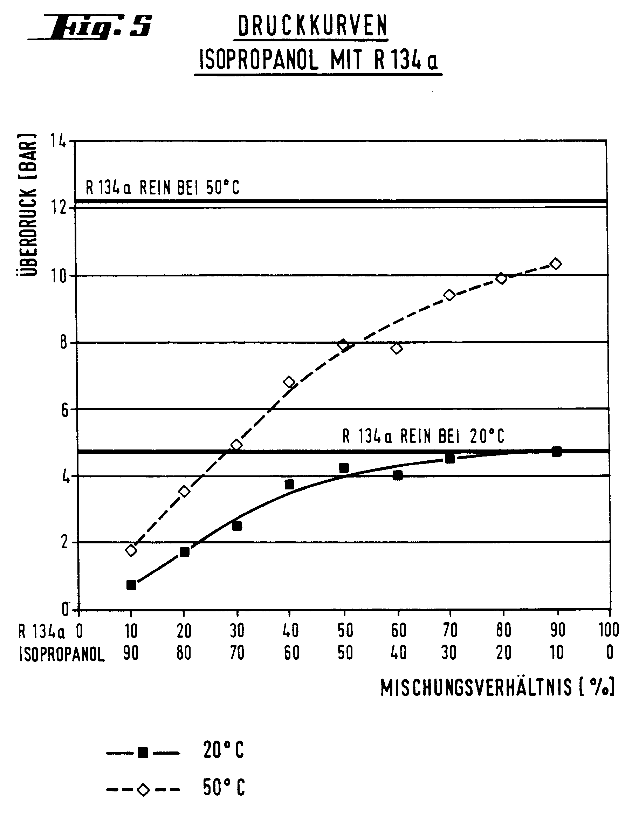

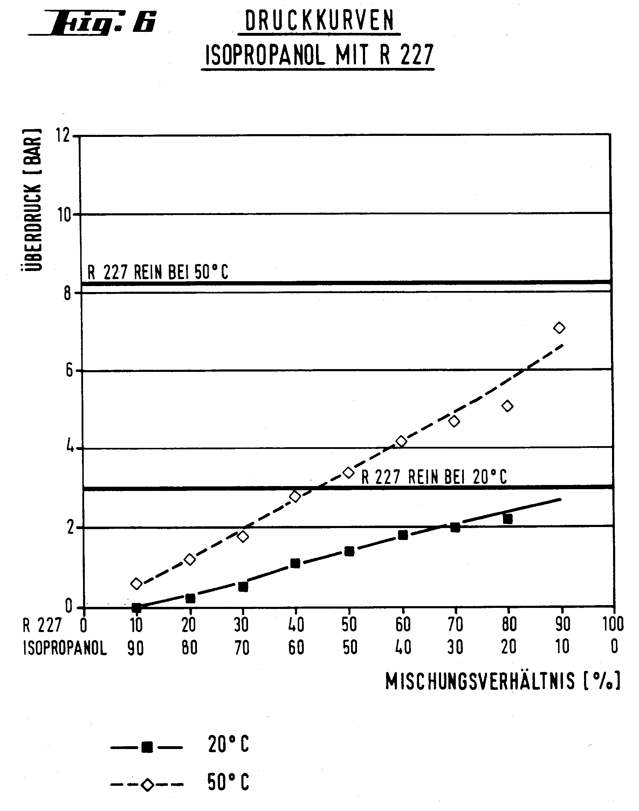

Es wurden jeweils Ethanol (99,8%) oder Isopropanol in 175 ml Aluminiumdosen eingewogen, die evakuierten Dosen mit Hilfe des zugehörigen Präzisionsventil verschlossen und R 134a oder R 227 in variierenden Mengen unter Druck eingefüllt, um verschiedene Mischungsverhältnisse von Alkohol zu R 134a und Alkohol zu R 227 einzustellen. Der Überdruck wurde immer nach 1h Lagerung bei 20°C und 50°C Lagertemperatur gemessen. Die Ergebnisse sind in den folgenden Tabellen 2 bis 5 zusammengestellt und als Kurven in den Figuren 3 bis 6 gezeigt. In den Tabellen sind darüberhinaus auch die gemessenen Dichten angegeben.

Tabelle 2

| Ethanol Gew.-% | R 134a Gew.-% | Überdruck (bar) | Dichte (kg/l) | Überdruck (bar) | Dichte (kg/l) |
|---|---|---|---|---|---|
| | | bei 20°C | | bei 50°C | |
| 0 | 100 | 4,7 | 1,226 | 12,2 | 1,103 |
| 10 | 90 | 4,3 | 1,182 | 10,9 | 1,069 |
| 20 | 80 | 4,2 | 1,139 | 10,4 | 1,035 |
| 30 | 70 | 4,0 | 1,095 | 9,8 | 1,001 |
| 40 | 60 | 3,7 | 1,051 | 8,8 | 0,967 |
| 50 | 50 | 3,4 | 1,008 | 7,8 | 0,933 |
| 60 | 40 | 3,0 | 0,964 | 6,8 | 0,899 |
| 70 | 30 | 2,4 | 0,920 | 5,5 | 0,865 |
| 80 | 20 | 1,3 | 0,876 | 3,7 | 0,831 |
| 90 | 10 | 0,3 | 0,833 | 2,0 | 0,797 |
| 100 | 0 | 0 | 0,789 | 0 | 0,763 |

Tabelle 3

| Ethanol Gew.-% | R 227 | Überdruck (bar) | Dichte (kg/l) | Überdruck (bar) | Dichte (kg/l) |
|---|---|---|---|---|---|
| | | bei 20°C | | bei 50°C | |
| 0 | 100 | 3,4 | 1,417 | 8,1 | 1,265 |
| 10 | 90 | 3,0 | 1,354 | 7,1 | 1,215 |
| 20 | 80 | 2,8 | 1,291 | 6,5 | 1,165 |
| 30 | 70 | 2,5 | 1,229 | 5,9 | 1,114 |
| 40 | 60 | 2,2 | 1,166 | 5,2 | 1,064 |
| 50 | 50 | 1,9 | 1,103 | 4,2 | 1,014 |
| 60 | 40 | 1,4 | 1,040 | 3,3 | 0,964 |
| 70 | 30 | 0,9 | 0,977 | 2,4 | 0,914 |
| 80 | 20 | 0,5 | 0,915 | 1,6 | 0,863 |
| 90 | 10 | 0,1 | 0,852 | 0,7 | 0,813 |
| 100 | 0 | 0 | 0,789 | 0 | 0,763 |

Tabelle 4

| Isopropanol Gew.-% | R 134a Gew.-% | Überdruck (bar) | Dichte (kg/l) | Überdruck (bar) | Dichte (kg/l) |
|---|---|---|---|---|---|
| | | bei 20°C | | bei 50°C | |
| 0 | 100 | 4,7 | 1,226 | 12,2 | 1,103 |
| 10 | 90 | 4,7 | 1,182 | 10,3 | 1,069 |
| 20 | 80 | 4,7 | 1,138 | 9,9 | 1,034 |
| 30 | 70 | 4,5 | 1,094 | 9,4 | 1,000 |
| 40 | 60 | 4,0 | 1,050 | 7,9 | 0,966 |
| 50 | 50 | 4,2 | 1,006 | 7,9 | 0,932 |
| 60 | 40 | 3,7 | 0,961 | 6,8 | 0,897 |
| 70 | 30 | 2,5 | 0,917 | 4,9 | 0,863 |
| 80 | 20 | 1,7 | 0,873 | 3,5 | 0,829 |
| 90 | 10 | 0,7 | 0,829 | 1,8 | 0,794 |
| 100 | 0 | 0 | 0,785 | 0 | 0,760 |

Tabelle 5

| Isopropanol Gew.-% | R 227 Gew.-% | Überdruck (bar) | Dichte (kg/l) | Überdruck (bar) | Dichte (kg/l) |
|---|---|---|---|---|---|
| | | bei 20°C | | bei 50°C | |
| 0 | 100 | 3,4 | 1,417 | 8,1 | 1,265 |
| 10 | 90 | 3,0 | 1,354 | 7,1 | 1,222 |
| 20 | 80 | 2,2 | 1,291 | 5,1 | 1,165 |
| 30 | 70 | 2,0 | 1,227 | 4,6 | 1,114 |
| 40 | 60 | 1,8 | 1,164 | 4,2 | 1,063 |
| 50 | 50 | 1,4 | 1,101 | 3,4 | 1,013 |
| 60 | 40 | 1,1 | 1,038 | 2,8 | 0,962 |
| 70 | 30 | 0,5 | 0,975 | 1,8 | 0,912 |
| 80 | 20 | 0,2 | 0,911 | 1,2 | 0,861 |
| 90 | 10 | 0 | 0,848 | 0,6 | 0,811 |
| 100 | 0 | 0 | 0,785 | 0 | 0,760 |

Wie den Druckwerten in den obigen Tabellen und den Figuren 3 bis 5 zu entnehmen ist, ist R 227 bereits ohne Zusätze für Aluminiumdosen (bis 12 bar) geeignet. Mit geringen zugesetzten Alkoholmengen wird ein Druck erreicht, der den Einsatz in Weißblechdosen möglich macht. Demgegenüber kann R 134a ohne Zusätze nicht verwendet werden. Für den Einsatz in Weißblechdosen sind hohe Zusätze an Alkoholen erforderlich.

Beispiel 3

In 80ml Aerosol-Glasflaschen wurden entsprechend dem gewünschten Mischungsverhältnis unterschiedliche Mengen der unten angegebenen oberflächenaktiven Stoffe mit Analysenwaage eingewogen und mit den zugehörigen AR-Ventilen (Deutsche Aerosol-Ventil GmbH) diffusionsdicht verschlossen. R 134a oder R 227 wurde mit Hilfe eines Adapters unter Gewichtskontrolle in die Glasflaschen gedrückt. Die Mischungen wurden anschließend 30 min geschüttelt, anschließend 12h ruhig bei Raumtemperatur stehen gelassen und dann auf eine eventuelle Abscheidung der oberflächenaktiven Stoffe geprüft und entsprechend beurteilt. Die Ergebnisse sind in Tabelle 6 zusammengestellt. Folgende oberflächenaktive Stoffe wurden untersucht:
OS 1 Polyoxyethylen(20)sorbitan-monolaurat (Tween 20[R])
OS 2 Polyoxyethylen(20)sorbitan-monooleat (Tween 80[R])
OS 3 Sorbitan-trioleat (Span 85[R])
OS 4 Glycerin-fettsäureester Arlacel 186[R]
OS 5 Ölsäure
OS 6 Benzododeciniumchlorid
OS 7 Epikuron
Die getesteten Mischungen wurden folgendermaßen beurteilt:

| Ausfällung der oberflächenaktiven Stoffe in fester Form | AF |
|---|---|
| Abscheidung großer Tropfen | +++ |
| Abscheidung kleiner Tropfen | ++ |
| Abscheidung vereinzelter Tropfen | + |
| keine Abscheidung | o |

Tabelle 6

| Oberflächenaktiver Stoff | Treibmittel | Gew.-% oberflächenak. Stoff im Treibmittel | | | | | |
|---|---|---|---|---|---|---|---|
| | | 0,05 | 0,15 | 0,25 | 0,5 | 0,75 | 1,0 |
| OS 1 | R 134a | o | o | + | ++ | +++ | +++ |
| OS 1 | R 227 | o | o | o | o | o | o |
| OS 2 | R 134a | + | ++ | ++ | ++ | ++ | +++ |
| OS 2 | R 227 | o | o | o | o | + | ++ |
| OS 3 | R 134a | +++ | +++ | +++ | +++ | AF | AF |
| OS 3 | R 227 | + | ++ | ++ | +++ | +++ | AF |
| OS 4 | R 134a | + | ++ | +++ | +++ | AF | AF |
| OS 4 | R 227 | + | + | ++ | +++ | +++ | +++ |
| OS 5 | R 134a | ++ | ++ | +++ | +++ | AF | AF |
| OS 5 | R 227 | + | ++ | ++ | +++ | +++ | +++ |
| OS 6 | R 134a | | | AF | AF | | |
| OS 6 | R 227 | | | ++ | +++ | | |
| OS 7 | R 134a | | | AF | AF | | |
| OS 7 | R 227 | | | + | ++ | | |
| Diese Ergebnisse zeigen, daß R 227 ein besseres Lösevermögen für einige üblicherweise in medizinischen Aerosolen eingesetzte oberflächenaktive Stoffe hat als R 134a und somit stabilere Aerosolformulierungen ergibt. | | | | | | | |

Beispiel 4

Um die Stabilität der in Beispiel 3 mit den oberflächenaktiven Stoffen OS 1 und OS 2 hergestellten Aerosolformulierungen weiter zu untersuchen, wurden die entsprechenden Mischungen nach der zwölfstündigen Lagerung weiter bei 6°C und 23°C 12 h gelagert und dann beurteilt. Die Ergebnisse sind in folgender Tabelle 7 zusammengestellt.

Tabelle 7

| Oberflächenaktiver Stoff | Treibmittel | Lagertemp.(°C) | Gew.-% oberflächenak.Stoff in Treibm | | | | | |
|---|---|---|---|---|---|---|---|---|
| | | | 0,05 | 0,15 | 0,25 | 0,5 | 0,75 | 1,0 |
| OS 1 | R 134a | 6 | o | o | + | + | ++ | +++ |
| OS 1 | R 134a | 23 | o | o | + | ++ | +++ | +++ |
| OS 1 | R 227 | 6 | o | o | o | o | o | o |
| OS 1 | R 227 | 23 | o | o | o | o | o | o |

Beispiel 5

In diesem Beispiel wurde die Beständigkeit von Aerosolformulierungen getestet, die neben R 227 Ethanol enthalten (Gewichtsverhältnis R 227 zu Ethanol 4 : 1). Dazu wurden in 80ml Aerosol-Glasflaschen entsprechend dem gewünschten Mischungsverhältnis unterschiedliche Mengen einiger der oben definierten oberflächenaktiven Stoffe mit Analysenwaage eingewogen und Ethanol zugesetzt. Die Glasflaschen wurden mit den zugehörigen AR-Ventilen diffusionsdicht verschlossen. R 227 wurde anschließend mit einem Adapter unter Gewichtskontrolle in die Glasflaschen gedrückt. Die Mischungen wurden anschließend 30 min geschüttelt und 12h lang ruhig stehengelassen und dann wie in Beispiel 3 beschrieben beurteilt. Die Ergebnisse in der folgenden Tabelle 8 zeigen, daß durch den Ethanolzusatz das Lösevermögen für die getesteten oberflächenaktiven Stoffe verbessert wurde und somit die Stabilität erhöht wird.

EP 0 550 031 B1

Tabelle 8

| Oberflächenaktiver Stoff | Gew.-% oberflächenaktiver Stoff in R 227 | | | | | |
|---|---|---|---|---|---|---|
| | 0,05 | 0,15 | 0,25 | 0,5 | 0,75 | 1,0 |
| OS 1 | o | o | o | o | o | o |
| OS 2 | o | o | o | o | o | o |
| OS 3 | o | o | o | + | + | + |
| OS 4 | o | o | o | o | o | o |
| OS 5 | o | o | o | o | o | o |

**Patentansprüche**

1. Medizinische Aerosolformulierung, die wenigstens einen medizinischen Wirkstoff, druckverflüssigtes Heptafluorpropan sowie wenigstens einen oberflächenaktiven Stoff, ausgenommen monoacetylierte oder diacetylierte Monoglyceride, enthält.

2. Medizinische Aerosolformulierung nach Anspruch 1, dadurch gekennzeichnet, daß sie zusätzlich eine Verbindung mit einer höheren Polarität als Heptafluorpropan enthält.

3. Medizinische Aerosolformulierung nach Anspruch 2, dadurch gekennzeichnet, daß die Verbindung mit einer höheren Polarität als Heptafluorpropan ein Alkohol ist.

4. Medizinische Aerosolformulierung nach Anspruch 3, dadurch gekennzeichnet, daß der Alkohol Ethanol ist.

5. Medizinische Aerosolformulierung nach Anspruch 3, dadurch gekennzeichnet, daß der Alkohol Isopropanol ist.

6. Medizinische Aerosolformulierung nach einem der Ansprüche 2 bis 5, dadurch gekennzeichnet, daß das Gewichtsverhältnis von Heptafluorpropan zu der Verbindung mit der höheren Polarität so gewählt ist, daß der Druck in dem die Aerosolformulierung enthaltenden Druckbehälter mindestens 3 bar bei Raumtemperatur beträgt.

7. Medizinische Aerosolformulierung nach einem der Ansprüche 3 bis 5, dadurch gekennzeichnet, daß das Gewichtsverhältnis von Heptafluorpropan zu dem Alkohol mindestens 70 : 30 beträgt.

8. Medizinische Aerosolformulierung nach Anspruch 7, dadurch gekennzeichnet, daß das Gewichtsverhältnis von Heptafluorpropan zu dem Alkohol im Bereich von 85 : 15 bis 96 : 4 liegt.

9. Medizinische Aerosolformulierung nach einem der Ansprüche 1 bis 8, dadurch gekennzeichnet, daß sie zusätzlich ein Treibmittel aus der Gruppe $C_aH_bF_c$ enthält, wobei $b + c = 2a + 2$ ist, und a 1 bis 3, b 0 bis 4 und c 2 bis 8 bedeutet.

10. Medizinische Aerosolformulierung nach Anspruch 9, dadurch gekennzeichnet, daß sie 50 bis 95 Gew.-% Heptafluorpropan enthält.

11. Medizinische Aerosolformulierung nach einem der Ansprüche 1 bis 10, dadurch gekennzeichnet, daß sie 2 bis 5 Gew.-% des medizinischen Wirkstoffs enthält.

12. Medizinische Aerosolformulierung nach einem der Ansprüche 1 bis 11, dadurch gekennzeichnet, daß sie 2-Hydroheptafluorpropan enthält.

**Claims**

1. A medicinal aerosol formulation which contains at least one medicinal active ingredient, heptafluoropropane liquefied under pressure and at least one surface-active substance, with the exception of monoacetylated or diacetylated monoglycerides.

2. The medicinal aerosol formulation as claimed in claim 1, which additionally contains a compound of higher polarity than heptafluoropropane.

3. The medicinal aerosol formulation as claimed in claim 2, wherein the compound of higher polarity than heptafluoropropane is an alcohol.

4. The medicinal aerosol formulation as claimed in claim 3, wherein the alcohol is ethanol.

5. The medicinal aerosol formulation as claimed in claim 3, wherein the alcohol is isopropanol.

6. The medicinal aerosol formulation as claimed in one of claims 2 to 5, wherein the weight ratio of heptafluoropropane to the compound of higher polarity is chosen so that the pressure in the pressurized container holding the aerosol formulation is at least 3 bar at room temperature.

7. The medicinal aerosol formulation as claimed in one of claims 3 to 5, wherein the weight ratio of heptafluoropropane to the alcohol is at least 70:30.

8. The medicinal aerosol formulation as claimed in claim 7, wherein the weight ratio of heptafluoropropane to the alcohol is in the range from 85:15 to 96:4.

9. The medicinal aerosol formulation as claimed in one of claims 1 to 8, which additionally contains a propellant selected from the group $C_aH_bF_c$, in which $b + c = 2a + 2$, and a is 1 to 3, b is 0 to 4 and c is 2 to 8.

10. The medicinal aerosol formulation as claimed in claim 9, which contains 50 to 95% by weight of heptafluoropropane.

11. The medicinal aerosol formulation as claimed in one of claims 1 to 10, which contains 2 to 5% by weight of the medicinal active ingredient.

12. The medicinal aerosol formulation as claimed in one of claims 1 to 11, which contains 2-hydroheptafluoropropane.

**Revendications**

1. Formulation médicale en aérosol, qui contient au moins une substance active médicinale, de l'heptafluoropropane liquéfié sous pression et au moins un agent tensioactif, à l'exception des monoglycérides monoacétylés ou diacétylés.

2. Formulation médicale en aérosol selon la revendication 1, caractérisée en ce qu'elle contient en outre un composé plus polaire que l'heptafluoropropane.

3. Formulation médicale en aérosol selon la revendication 2, caractérisée en ce que le composé plus polaire que l'heptafluoropropane est un alcool.

4. Formulation médicale en aérosol selon la revendication 3, caractérisée en ce que l'alcool est l'éthanol.

5. Formulation médicale en aérosol selon la revendication 3, caractérisée en ce que l'alcool est l'isopropanol.

6. Formulation médicale en aérosol selon l'une des revendications 2 à 5, caractérisée en ce que le rapport en masse de l'heptafluoropropane au composé plus polaire est choisi de façon que la pression dans le récipient sous pression contenant la formulation en aérosol soit d'au moins 3 bars à la température ambiante.

7. Formulation médicale en aérosol selon l'une des revendications 3 à 5, caractérisée en ce que le rapport en masse de l'heptafluoropropane à l'alcool est d'au moins 70 : 30.

8. Formulation médicale en aérosol selon la revendication 7, caractérisée en ce que le rapport en masse de l'heptafluoropropane à l'alcool est compris entre 85 : 15 et 96 : 4.

9. Formulation médicale en aérosol selon l'une des revendications 1 à 8, caractérisée en ce qu'elle contient en outre un gaz propulseur du groupe de $C_aH_bF_c$, où $b + c = 2a + 2$, et a est un nombre de 1 à 3, b un nombre de 0 à 4 et c un nombre de 2 à 8.

**10.** Formulation médicale en aérosol selon la revendication 9, caractérisée en ce qu'elle contient 50 à 95 % en masse d'heptafluoropropane.

**11.** Formulation médicale en aérosol selon l'une des revendications 1 à 10, caractérisée en ce qu'elle contient 2 à 5 % en masse de la substance active médicinale.

**12.** Formulation médicale en aérosol selon l'une des revendications 1 à 11, caractérisée en ce qu'elle contient du 2-hydroheptafluoropropane.

**Fig. 1** DICHTE VON FLÜSSIGKEITEN
d = f(T)

—•— R 227
—+— R 134a
—×— R 11/12 (50:50)
—□— R 12/114 (40:60)

# _Fig. 2_   GASDRÜCKE

$$p = f(T)$$

Legend:
- —•— R 227
- —+— R 134 a
- —×— R 11/12 (50:50)
- —□— R 12/114 (40:60)
- —△— R 12

**Fig. 3**

DRUCKKURVEN
ETHANOL(99,8%)MIT R134a

**Fig. 4** DRUCKKURVEN ETHANOL (99,8%) MIT R 227

Fig: 5

DRUCKKURVEN
ISOPROPANOL MIT R 134 a

*Fig. 6*   DRUCKKURVEN
ISOPROPANOL MIT R 227